# EUROPEAN PATENT SPECIFICATION

(11) **EP 1 795 115 B1**
(45) Date of publication and mention of the grant of the patent: **13.04.2016**
(21) Application number: 05027089.1
(22) Date of filing: 12.12.2005
(51) Int. Cl.: A61B 1/01, A61B 1/31, A61M 25/00

(54) **Endoscope having guiding means with variable stiffness**
Endoskop mit Führungsmitteln mit verstellbarer Steifigkeit
Endoscope avec des moyens de guidage à rigidité variable

(43) Date of publication of application: 13.06.2007
(73) Proprietor: Invendo Medical GmbH, 69469 Weinheim (DE)
(72) Inventor: Bob, Konstantin, 69469 Weinheim (DE); Pauker, Fritz, 86438 Kissing (DE); Viebach, Thomas, 82282 Pischertshofen (DE)
(74) Representative: Winter, Brandl, Fürniss, Hübner, Röss, Kaiser, Polte - Partnerschaft mbB

(56) References cited:
- EP-A- 1 203 564
- EP-A- 1 654 976
- WO-A-03/073921
- DE-A1- 19 729 499
- US-A- 4 815 450
- US-A- 4 893 613
- US-A1- 2004 186 350

## Description

The invention relates to an endoscope in accordance with the preamble of claim 1.

Endoscopes have become an important aid in technology and above all in medicine to inspect canal-shaped cavities which otherwise are only accessible by considerable operations. Endoscopes are preferably used for examining the esophagus, the stomach, the duodenum from the stomach, the intestines from the anus, the urethra, the bladder and the ureter.

Such endoscopes are equipped at their leading end with a lighting means and with an optical system for visually detecting the area upstream of the cavity. The optical information detected at the leading end of the endoscope usually is either transmitted to the rear by means of fiber optics through the endoscope to its operating end or is detected by means of an optical sensor chip at the leading end, is guided to the rear via an electric line through the endoscope and is displayed on a screen. Moreover also a transmission of information via radio from the leading end to the operating end is possible.

Furthermore, endoscopes usually comprise a working conduit, as it is called, through which various operating instruments can be introduced and operated. For instance, small forceps for taking tissue specimen, biopsy needles, heatable cutting wires, small scissors, coagulation electrodes or the like are introduced to carry out surgical measures on damaged tissue where necessary. Finally a fluid conduit for wash and operating wires or fluid lines for bending the leading end of the endoscope in several directions are usually provided. These operating wires or fluid lines are in turn guided inside the endoscope shaft to the leading or distal end thereof.

For introducing and moving forward the endoscope shaft in the canal-shaped cavity to be inspected DE 42 44 990 discloses a respective device. In this device a slip hose is moved along with the endoscope shaft during the forward movement of the endoscope shaft into a cavity and is slipped inside-out upon reaching the distal end so that the portion of the slip hose slipped inside-out is resting vis-à-vis the cavity wall. The advantage of this resting portion of the slip hose consists in the fact that damages of the cavity wall are reduced. Moreover, it has been assumed with this device that when applied in medical engineering the pain for the patient can be significantly reduced due to the forward movement when the slip hose is resting relative to the intestinal wall. However, recent examinations have shown that the principal reason for the pain during coloscopy is not the relative movement between the introduced endoscope shaft and the intestinal wall, but that the pain is mainly caused by pressing the distal end of the endoscope shaft against the curved outer wall of intestinal bends. (In this context, each of the terms curved outer wall and inner wall denotes the cavity wall lying further outside or inside with respect to a fictitious center of a cavity bend.).

WO 03/073921 A discloses an assembly for advancing a colonoscope through the colon featuring guiding means and means of variable stiffness.

Therefore it is an object of the invention to realize an endoscope comprising a propulsion system of a simple design by which less pressure is exerted against the curved outer wall.

### SUMMARY OF THE INVENTION

This object is achieved by an endoscope including the features of claim 1.

Further advantageous configurations of the invention are the subject matter of the other claims.

### BRIEF DESCRIPTION OF THE DRAWINGS

Hereinafter illustrative embodiments are described, reference to not all of which are part of the invention. This is done with the enclosed drawings, the following being schematically illustrated in the reference to the enclosed drawings, the following being schematically illustrated in the drawings:
Fig. 1 is a spatial representation of an endoscope.
Fig. 2 shows drive means for moving an endoscope forward.
Figures 3A to 3C are front views of an endoscope illustrating the progressive movement of the endoscope.
Fig. 4 is a spatial representation of an endoscope according to a first embodiment of the present invention.
Figures 5A to 5C are front views of the endoscope illustrating the progressive movement of an endoscope.
Fig. 6 shows another endoscope.
Fig. 7 shows a modification of the first embodiment.
Figs. 8(a) and 8(b) show cross-sectional views of the endoscope shaft of figure 7.

### DETAILED DESCRIPTION OF THE PREFERRED EMBODIMENTS

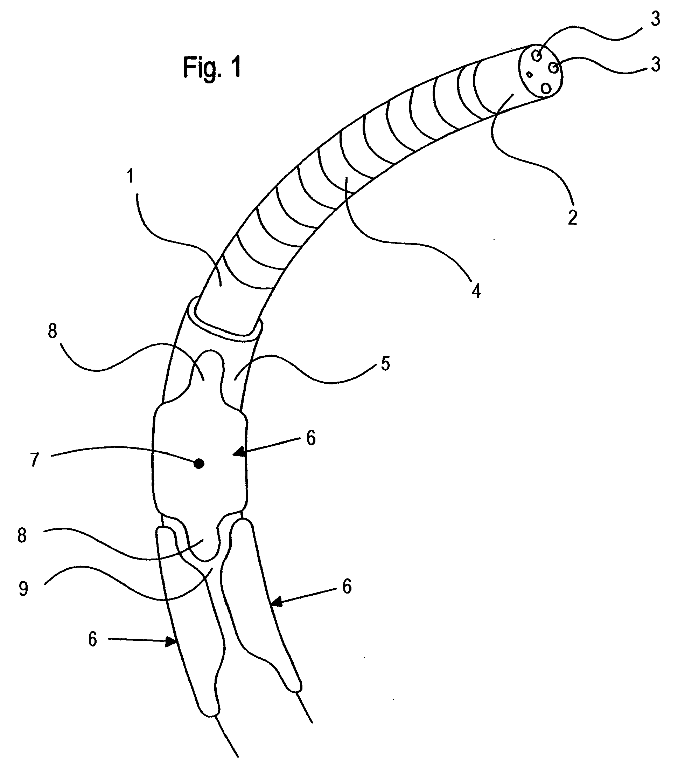

By way of the Figures 1 to 3C an endoscope with alternating propulsion is described in detail below. In accordance with these figures an endoscope comprises a flexible endoscope shaft 1 to the distal end of which an endoscope head 2 is attached and to the other end (operating end) of which operating devices and, where appropriate, information evaluating devices (not shown) are connected. This endoscope head 2 may include various means 3, such as for instance an optical system, a lighting means, a working conduit for different tools etc.

The endoscope head 2 is preferably connected to the flexible endoscope shaft 1 by a movable distal end portion 4 (hereinafter referred to as "deflecting"). The deflecting 4 can be bent in several radii of curvature, preferably in several directions, in response to the instructions from the operating end (not shown) of the endoscope. Here a conventional deflecting is employed, as it is described, for instance, in the applications DE 102 09 986 A1 or DE 100 10 932 A1. Concerning the exact functioning and design of this deflecting reference is made to these applications. In this context, it is merely stated that, as already mentioned, the deflecting can be bent by control signals and/or pressures from the operating end and thus can also be optionally stiffened in this bent position or in a not bent position.

The lines (not shown) for controlling the deflecting 4 and the means 3 as well as possibly provided working conduits for operating and introducing tools used at the endoscope head 2 extend inside the deflecting 4 and the endoscope shaft 1 backwards to the operating end.

The endoscope shaft 1 is preferably surrounded by a preferably single-wall hose 5 up to the position at which the endoscope shaft 1 is connected to the deflecting 4 so that the deflecting 4 including the endoscope head 2 connected thereto projects from the leading end (distal end) of the hose 5. In some cases the hose consists of EPTFE material having a wall thickness of 2 to 3 mm. For reinforcement a spiral spring can be introduced in the hose-shaped EPTFE material such that the EPTFE hose material extends conically with respect to the spiral spring and the spring wire (not necessarily made of spring steel) is completely embedded in EPTFE material between the inner hose wall and the outer hose wall. Moreover, it is also possible to arrange the spiral spring conically with respect to the EPTFE material, wherein the spiral spring is enclosed with the EPTFE hose material and is connected to the same so that the spiral spring is exposed to the inside. Furthermore, the spiral spring is not absolutely necessary and a design of the hose 5 without spiral spring is imaginable, too. The hose material is not restricted to said EPTFE material, either, and may be made of other materials such as silicone, for example.

The hose 5 can be provided with fluid pads 6 at the outer circumference. These fluid pads 6 preferably have an oblong design and are broadened at the central portions 7 thereof. Thus the fluid pads 6 have at their leading end and their trailing end a narrow tongue-shaped end portion 8.

The fluid pads 6 are arranged in groups, each consisting of three fluid pads 6. The fluid pads 6 of this group of three are aligned in parallel to each other about the outer circumference of the hose 5 so that the side faces of the fluid pads 6 contact each other at their central portion 7 or form a small clearance. That is to say, this group of three fluid pads is distributed around the outer circumference of the hose such that the mean perpendiculars of the fluid pads 6 intersect the central axis of the endoscope shaft 1 and form an angle of 120° with respect to the mean perpendicular of the respective neighboring fluid pad 6. Thus, in this group the tongue-shaped end portions 8 project at a distance of 120° (in a plane normal to the central hose axis) to the front and to the rear distributed around the outer circumference of the hose 5. Since the end portions 8 are narrower than the central portions 7, an end portion is further spaced apart from the respective neighboring end portion than the respective central portions 7. In this way bulges 9 are formed between two respective neighboring end portions 8.

Adjacent to the above-mentioned group of three fluid pads 6 another group of three fluid pads 6 is arranged ahead of and behind the former in such manner that the end portions 8 of the latter group of three fluid pads 6 are inserted in the bulges of the former group of three fluid pads 6. That means that fluid pad groups adjacent in the longitudinal direction are offset by 60° in the circumferential direction in a plane normal to the central hose axis.

The fluid pads 6 are arranged in accordance with the afore-described way approximately along an end portion of 50 cm in length of the hose 5 starting from the leading end thereof.

Pressure has to be applied to the fluid pads 6 via feed lines (not shown) extending in the wall or at the inner or outer circumference of the hose 5 backwards to the operating end.

Depending on the pressure applied to the fluid pads 6 the flexibility of the hose 5 can be varied. Moreover, the air pads 6 get in contact with the wall of the cavity so that the hose 5 is stationary vis-à-vis the wall of the cavity. Thus, the application of pressure to the fluid pads 6 and/or a relief of incompressible fluid in the fluid pads 6 permits to optionally impart flexibility to the hose 5 which is lower or higher than the flexibility of the endoscope shaft 1 as well as to simultaneously put the hose 5 into a stationary or movable condition vis-à-vis the cavity wall.

Due to the fact that the air pads overlap at the tongue-shaped end portions 8, the hose 5 can be supported at the entire circumference of the cavity wall without loosing its flexibility.

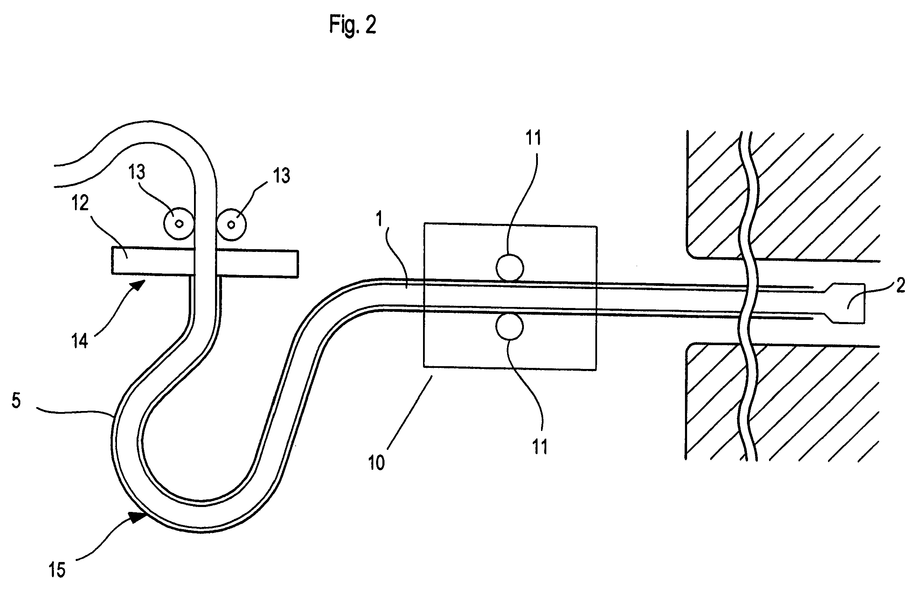

Fig. 2 shows the endoscope in a state in which it has already been moved a bit into the cavity. Reference numeral 10 denotes a first drive means 10 for a progressive movement of the hose 5. The drive means 10 comprises a pair of drive wheels 11 which are frictionally engaged from two sides with the hose 5 by their groove-shaped outer circumference. These drive wheels 11 are driven by an electric motor which is not shown. The hose 5 is fastened to a plate-shaped member 12 by its trailing end. Between the first drive means 10 and the plate 12 the endoscope shaft 1 is laid with the surrounding hose 5 into a bulged shape 15. In the area of the plate 12 lubricating stuff might be pressed into the space between the endoscope shaft 1 and the hose 5.

At the side of the plate 12 opposed to the hose 5 preferably a pair of drive wheels 13 is provided which are frictionally engaged from two sides with the endoscope shaft 1. These drive wheels 13 form a second drive means 14 together with an electric motor, which is not shown, and the plate 12 connected to the drive wheels 13.

The first drive means 10 is fixedly connected to the second drive means 14 so that these drive means cannot move relative to each other. Moreover, these drive means 10, 14 have to be arranged with respect to the cavity to be examined such that they do not move relative to the cavity.

The surface of the drive wheels 11 at their groove-shaped outer circumference is designed such that great friction occurs between the drive wheels 11 and the hose 5. Between the hose 5 and the endoscope shaft 1 either lubricating stuff is introduced and/or the endoscope shaft 1 is coated (e.g. Teflon) such that a minimal friction occurs between the hose 5 and the endoscope shaft 1. Thus it is possible that the hose 5 is frictionally driven by means of the drive wheels 11, while the endoscope shaft 1 is moved into the opposite direction by the drive wheels 13 or is at rest.

### (Functioning of the above endoscope)

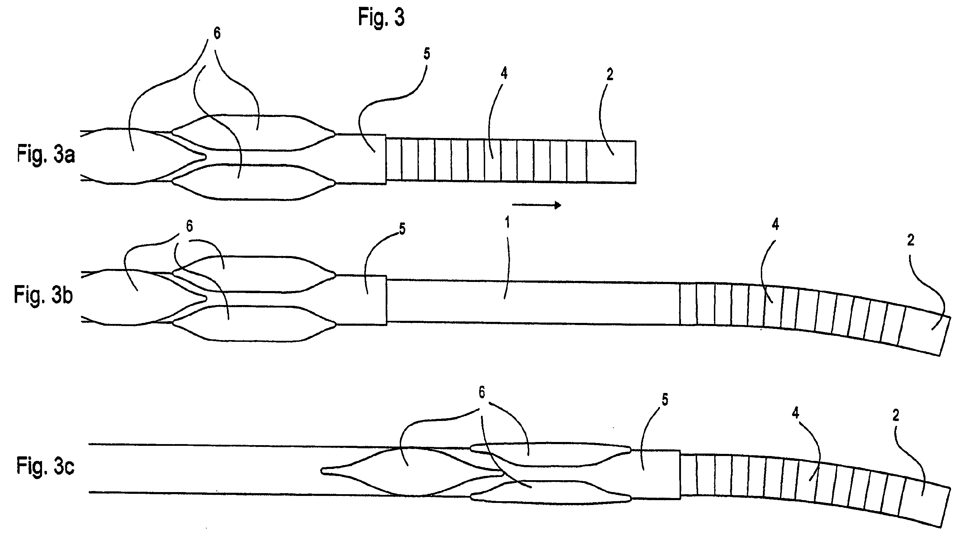

The functioning of the above endoscope is described with reference to Figures 2 and 3.

In Figure 3a the endoscope shaft 1 and the hose 5 are provided at a particular position of a cavity to be inspected. The fluid pads 6 are filled with fluid by the pump (not represented) and thus get into contact with the cavity wall. Due to the pressure of the fluid pads 6 against the cavity wall the hose 5 is stationary with respect to the cavity wall. In addition or alternatively, the hose 5 is held by the drive means 10. Moreover the fluid supply is controlled in such manner that the hose 5 has a lower flexibility (higher stiffness) than the endoscope shaft 1 when the filling of the fluid pads is completed.

Based on this state, the endoscope shaft 1 is moved forward by means of the second drive unit 14. The first drive unit 10 is idling. This operation is schematically represented in Figure 3b. When the endoscope shaft 1 is moved forward, the hose 5 serves as a supporting conduit for the movement of the endoscope shaft 1. This supporting effect is assisted by the lower flexibility of the hose 5.

If it is necessary during this progressive movement of the endoscope shaft 1 to guide the front portion of the endoscope shaft 1 along a bend of the cavity, it is proceeded as follows. First of all the cavity bend can be detected at the endoscope head 2 by sensors or by the optical system installed in the endoscope head. When the endoscope head 2 is moved forward along the bend of the cavity, the curvature of the deflecting 4 can be adjusted and adapted to the radius of curvature of the cavity bend. Thus, no pressure or only low pressure is exerted on the curved outer wall of the cavity. When the endoscope head 2 has reached an end of the cavity bend, the deflecting 4 is straightened upon the further forward movement of the endoscope head 2.

When the endoscope shaft 1 has been moved out of the hose 5 (has advanced) over a particular predetermined distance by means of the second drive unit 14, the second drive unit is stopped, whereby the shaft 1 is retained.

The further progressive movement is shown in Figure 3c. Accordingly, the fluid pads 6 are relieved so that the hose 5 is movable with respect to the cavity wall. Moreover, when the fluid pads 6 are relieved the hose 5 has a higher flexibility than the endoscope shaft 1. In this state the hose 5 is moved forward by the first drive means 10. During this progressive movement the bulged shape 15 of endoscope shaft 1 and hose 5 becomes smaller. By the fact that the drive means 14 retains the endoscope shaft 1 it is obtained that the endoscope shaft 1 is resting with respect to the cavity to be inspected, while the leading end of the hose 5 moves in the direction of the leading end of the endoscope shaft 1 (concerning the progressive movement of the hose 5 several modifications are possible which are described at the end of this description).

During this progressive movement of the hose 5 the endoscope shaft 1 resting with respect to the cavity acts with its lower flexibility (higher basic stiffness compared to that of the hose 5) as a rail for the hose 5. Since the endoscope shaft 1 is provided further inside the cavity than the hose 5, at the time when the hose 5 starts moving the endoscope shaft 1 has already adopted the shape of the cavity, through which the hose 5 passes in one cycle, with all possible bends. When, thus, the hose 5 follows the shape of the endoscope shaft 1, the force by which the hose 5 presses against the curved outer wall can be reduced. In the present invention, the force otherwise pressing against the curved outer wall is deflected by the endoscope shaft 1 which is stiffer in this state. For use in medicine this means at the same time that the pain felt by the patient does not occur or is reduced.

Preferably the hose 5 is moved forward, as represented in Figure 3c, until the leading end of the hose 5 reaches the connecting point between the hose 5 and the deflecting 4. After completion of such a cycle of movement as described by way of the Figures 3a to 3c, the progressive movement of the endoscope is continued in a new cycle starting with Figure 3a. That is to say, the fluid pads 6 are filled again so that the hose 5 is stationary relative to the cavity wall. Subsequently the endoscope shaft 1 is moved forward etc.

This alternating propulsion of the endoscope shaft 1 and the hose 5 is repeated until the desired location to be examined in the cavity is reached.

The endoscope can be moved backward by reversing the course of the forward movement. Another possibility would be to relieve the fluid pads and to release the deflecting 4 such that it is freely movable. After that the endoscope shaft could be withdrawn from the cavity together with the hose 5.

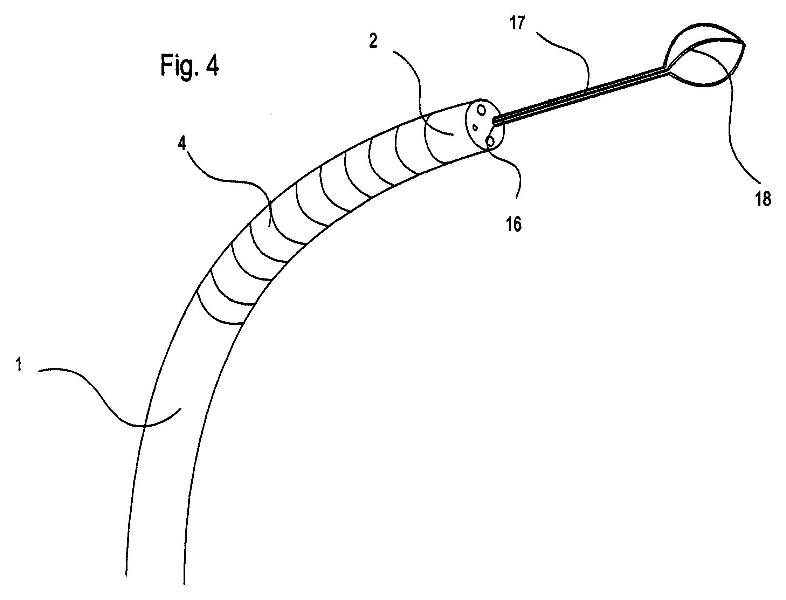

Figure 4 shows another design for an endoscope representing a first embodiment of the invention. In the description of this embodiment components which are equal to those of the above described endoscope are denoted with the same reference numerals and the description of these components is omitted.

In this embodiment no hose including air pads is provided, instead a guiding wire 17 having a lower flexibility than the endoscope shaft 1 is guided through the working conduit 16 of the endoscope shaft 1. The guiding wire 17 is connected at its leading end to a foldable cage 18. This cage 18 can be guided through the working conduit 16 in the folded state.

When introducing the endoscope shaft 1 into a cavity to be inspected, the cage 18 and some centimetres of the guiding wire 17 project forward from the leading end of the endoscope shaft 1. Then the cage 18 is introduced visibly a bit into the cavity. Subsequently the endoscope shaft 1 is advanced by a drive or manually, while the guiding wire 17 including the cage 18 is brought into a rest position vis-à-vis the cavity, for instance by holding the rear end of the guiding wire 17 in place.

During the forward movement of the endoscope shaft 1 the guiding wire 17 resting vis-à-vis the cavity serves as a rail for the endoscope shaft 1. Thus, the endoscope shaft 1 follows in its forward movement the guiding wire 17 and, in the case of a bend of the cavity, is guided along the bend without applying too high pressure to the curved outer wall. By virtue of the lower flexibility of the guiding wire 17 compared to the flexibility of the endoscope shaft 1, the forces which otherwise would be applied by the endoscope shaft 1 to the curved outer wall are deflected via the stiffer guiding wire 17.

When the endoscope shaft 1 has approached the rear end of the cage 18, the propulsion of the endoscope shaft 1 is stopped and the guiding wire 17 including the cage 18 is moved forward by means of a drive or manually, while the endoscope shaft 1 remains in a resting position vis-à-vis the cavity.

Due to the shape of the cage 18 tapered to the front, the cage can be guided along the cavity and along cavity bends without pressing too strongly against a curved outer wall and instead it follows the bend of the cavity. During its forward movement the guiding wire 17 is supported by the endoscope shaft 1. If the guiding wire 17 has been moved out of the endoscope shaft over a predetermined distance so that the cage 18 has moved forward by such predetermined distance from the leading end of the endoscope shaft 1, again the guiding wire 17 including the cage 18 is brought into a resting position vis-à-vis the cavity, while the endoscope shaft 1 is moved forward using the guiding wire 17 as a rail, etc.

This alternating propulsion of the endoscope shaft 1 and the guiding wire 17 including the cage 18 is repeated until the desired location to be examined in the cavity is reached.

Optionally the shaft or the guiding wire can be provided with temporary stiffening means as they are mentioned in the introductory part of the specification and/or as modifications.

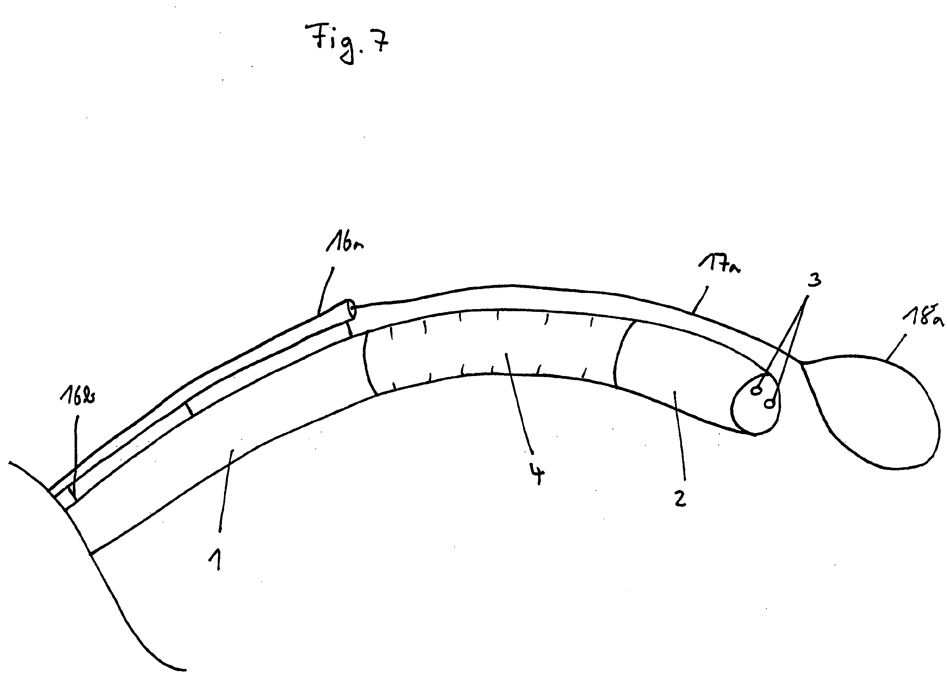

In a modification of the aforementioned embodiment according to the invention a continuous drive type endoscope is provided which is shown schematically in figure 7.

It is noted that the description of parts which have been described in detail above will be omitted. These parts are designated with the same reference numerals as above.

In this modification the endoscope comprises at least an endoscope shaft 1 (which might comprise the same means as in the aforementioned embodiments) at the outer circumference of which a catheter hose 16a acting as stiffness adjusting means and as guiding means is disposed in the longitudinal direction and/or in the axial direction of the endoscope shaft 1. Preferably plural catheter hoses 16a are disposed evenly, i.e. in equal and/or regular angular distances with respect of the circumferential direction of the endoscope shaft 1 along the circumference to the endoscope shaft 1. In a preferred formation of the endoscope shaft 1, exactly three catheter hoses 16a are disposed in the above manner at the outer circumference of the endoscope shaft 1 at angles of 120°.

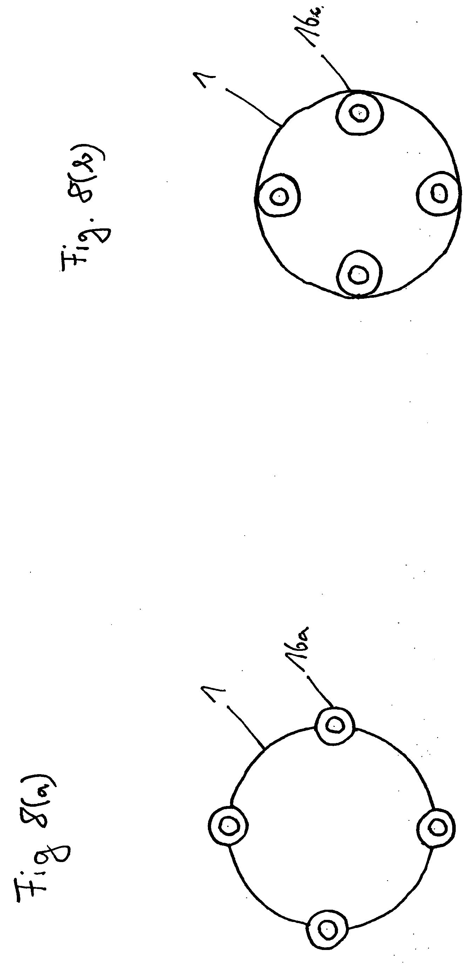

However, the catheter hoses 16a do not necessarily have to be disposed at the outer circumference of the endoscope shaft 1. As it can be gathered from the figures 8 (a) and 8 (b), the endoscope shaft 1 is shown in cross section, i.e. a sectional view in a plane perpendicular to the longitudinal direction or axial direction of the endoscope shaft 1 is given.

In figure 8 (a), the catheter hoses 16a are disposed n grooves which are formed on the outer circumference of the endoscope shaft 1 in the longitudinal direction thereof so that only a part of the catheter hoses 16a protrudes over the outer circumference of the endoscope shaft 1 in the radial direction thereof.

Figure 8 (b) shows another cross section of the endoscope shaft 1. Instead of disposing the catheter hoses 16a at the outer circumference or grooves of the endoscope shaft 1, canals 16c are formed in the interior of the endoscope shaft 1 which might be formed by catheter hoses 16a but do not have to and extend in the longitudinal direction thereof and are preferably disposed close to the outer circumference of the endoscope shaft 1.

The endoscope shaft 1 is extremely flexible, as mentioned in the foregoing. However, it is necessary in this embodiment that it takes up mainly forces acting in a radial direction of the endoscope shaft 1. Such forces are due to the arrangement of the catheter hose 16a. Therefore, preferably a wire spiral (not shown in figure 7) which only insignificantly impairs the bending capability of the endoscope shaft 1 but which permits taking up forces in the radial direction of the endoscope shaft 1 is incorporated in the endoscope shaft 1 in the longitudinal direction of the latter. It is noted that other devices known in prior art which permit said take-up of forces in the radial direction of the endoscope shaft 1 may equally be used.

The catheter hose 16a acting as stiffness adjusting means and as guiding means is capable of varying the stiffness of the same. Accordingly, the endoscope shaft 1 can be prevented from being bended excessively by varying the stiffness, as it will be explained in detail below. This may be effected by methods already known in prior art, as they have been mentioned, inter alia, in the foregoing.

The catheter hose 16a shown in figure 7 is mounted on the endoscope shaft 1 by means of supports 16b which may be known from prior art. Preferably the supports 16b hold the catheter hose 16a such that a distance and/or clearance is formed in the radial direction of the endoscope shaft 1 between the catheter hose 16a and the endoscope shaft 1, which distance or clearance is preferably within a range of from 0.5 cm to 1 cm. Thus, no relative movement of the catheter hose 16a with respect to the endoscope shaft 1 is possible (which is also the case in formations shown in figures 8 (a) and 8 (b)). Preferably, the supports 16b are elastically deformable in the radial direction of the endoscope shaft 1, wherein the degree of deformation is adjustable by the choice of the respective supports 16b.

However, these supports 16b do not necessarily have to be arranged between the endoscope shaft 1 and the catheter hose 16a. As mentioned above, it is also possible that grooves (not shown in figure 7 but in figure 8 (a)) are formed in the longitudinal direction of the endoscope shaft 1 in which the catheter hose 16a is embedded and fixedly arranged. Accordingly, the supports 16b can be omitted.

Also other attachments of the catheter hose 16bon the endoscope shaft 1 are possible as long as the catheter hose 16 is able to act as stiffness adjusting means and guiding means.

In a specific modification of the arrangement of the catheter hose 16a on the endoscope shaft 1, every respective catheter hose 16a is fixedly mounted on the endoscope shaft 1 via only one support 16b which is preferably disposed at the distal end of the endoscope shaft. The remaining part of the catheter hose 16a is guided in the longitudinal direction of the endoscope shaft 1 by at least one guide slide bearing or a sliding support which is fixedly arranged on the endoscope shaft 1 in its longitudinal direction. That is, in more detail, while the part of catheter hose 16b at the distal end of the endoscope shaft 1 is fixedly mounted on the endoscope shaft 1 via the only one support 16b and, thus, is not movable relative to the endoscope shaft 1, the other remaining part of the catheter hose 16a which leads to the proximal end of the endoscope shaft 1 and which is guided by the at least one guide slide bearing or the sliding support is movable in the longitudinal direction of the endoscope shaft 1 but not in the radial direction thereof and, thus, is movable relative to the endoscope shaft 1. Preferably, according to this modification, the endoscope shaft 1 as well as the catheter hose 16a have embedded or incorporated a spiral wire, additionally, other than the abovementioned spiral wire or any comparable means which allows them to take up forces in the longitudinal direction thereof. However also equivalent or comparable means known in the prior art can be used for the above described take-up of forces in the longitudinal direction of the endoscope shaft 1 as well as of the catheter hose 16a. That is, the endoscope shaft 1 does have to comprise means in this modification which are configured in such a manner that the endoscope shaft 1 has a flexural rigidity or flexural stiffness.

In the case of the catheter hoses 16a and 16c shown in figures 8 (a) and 8 (b), only the end portion of the respective catheter hose 16a, 16c is fixedly mounted to the endoscope shaft 1 at its distal end, for example, by an adhesive or the like so that the above-mentioned supports 16b can be omitted. Also in this modification, the endoscope shaft 1 as well as the catheter hose 16a have embedded or incorporated a spiral wire other than the above-mentioned spiral wire which allows them to take up forces in the longitudinal direction thereof. In other words, the endoscope shaft 1 has means which are configured such that the endoscope shaft has a flexural rigidity or flexural stiffness as mentioned above. Accordingly, in order to allow relative movement of the remaining part of the respective catheter hose 16a, 16c in the longitudinal direction of the endoscope shaft 1, a lubrication is provided between the above-mentioned remaining part of the catheter hose 16a, 16c and the groove or canal of the endoscope shaft in which the remaining part of catheter hose is disposed and guided. For example, the lubrication is provided by a lubricant like oil etc.

The guiding capability of the groove or the canal in which the respective catheter hose 16a, 16c is disposed may be enhanced by separate guiding means formed on the respective catheter hose 16a, 16c and the canal or groove. For example a guide rail might be provided formed on one of the respective catheter hose 16a, 16c and groove or canal, while a guiding groove suitable to fit and engage with the guiding rail is formed on the other of respective catheter hose 16a, 16c and groove or canal. However, any guiding means known in the prior art can be used.

Preferably, an elastic guiding wire 17a acting as guiding element is glidingly supported in the catheter hose 16a, thereby the guiding wire 17a being movable in the catheter hose 16a. At the end of the guiding wire 17a a loop 18a projecting from a distal end of the endoscope shaft 1, i.e. from a leading end of the endoscope shaft 1 with respect to the moving direction of the endoscope shaft 1 is formed. Thus, the loop 18a of the guiding wire 17a protrudes in the axial direction of the endoscope shaft beyond the distal end portion of the endoscope shaft 1 (also beyond the deflecting 4 and the endoscope head 2 of the endoscope shaft) by a predetermined distance which is variable by the frictional/sliding support. It is noted that the catheter hose 16a is only attached to the endoscope shaft 1 via the supports 16b regarding now the modification of fig. 7. The deflecting 4 and the endoscope head 2 correspond to a movable distal end portion of the endoscope shaft 1 which are not in contact with the catheter hose 16a or the supports 16b. The other end of the guiding wire 17a protrudes beyond the proximal end portion of the endoscope shaft 1 (which corresponds to the operating end of the endoscope shaft 1) by a predetermined length. As described hereinafter in the detailed functional description of the endoscope, the guiding wire 17a is operated by the end of the guiding wire 17a protruding from the proximal end portion of the endoscope shaft 1 for shifting the guiding wire 17a and thus for shifting the loop 18a provided at the distal end portion of the endoscope shaft 1. In an alternative embodiment of the loop 18a the dimension thereof, i.e. the size of the loop 18a is variable as in the aforementioned second embodiment (cf. the foldable cage 18 of the second embodiment).

The end of the guiding wire 17a projecting from the distal end portion of the endoscope shaft 1 may equally consist of plural loops 18a which are arranged with respect to each other such that a spherical or, respectively, ball-shaped or an ellipsoidal hollow body is formed. It is important in this context, as evident from the following, that by the round or curved shape of the loop 18a or the hollow body, respectively, the resistance during propulsion of the loop 18a or the hollow body with respect to the canal-shaped cavity to be examined is minimized. Furthermore, a cage provided at the end of the guiding wire 17a instead of the loop 18a or the hollow body is possible, as explained in the afore-described embodiment. However, as can be gathered from the functional description below, the loop 18a provided at the distal end of the guiding wire 17a does not necessarily have to be provided at the distal end of the guiding wire 17a. This loop 18a serves to assist during propulsion of the endoscope shaft 1.

The functioning or, rather, the drive mode of the continuous drive type endoscope in the canal-shaped cavity to be examined will be described hereinafter.

The endoscope is introduced into the canal-shaped cavity to be examined, preferably, by the guiding wire 17a projecting from the distal end portion of the endoscope shaft 1 or the loop 18a projecting from the distal end of the endoscope shaft 1. However, as long as the canal-shaped cavity comprises portions which are easy to pass, the guiding wire 17 or the loop 18a does not necessarily have to protrude beyond the distal end portion of the endoscope shaft 1. The endoscope is propelled by a drive means (not shown in fig. 7) which may be a conventional drive means for continuous drive type endoscopes known from prior art. The drive means may either drive the catheter hose 16a, which is fixedly arranged on the endoscope shaft, or the endoscope shaft 1. Likewise a drive of both aforementioned elements is possible. That is, in more concrete terms, the endoscope shaft 1 can be driven by the drive means as long as the stiffness thereof is sufficient for moving the endoscope shaft 1 in the canal-shaped cavity. If the stiffness thereof is or becomes insufficient, the catheter hose 16a is controlled such that it acts as guiding means and as stiffness adjusting means increasing the stiffness by methods known by the prior art in order to reinforce the endoscope shaft in the axial direction thereof. Preferably, the stiffness of the catheter hose 16a is adjusted by introducing an elastic wire having optionally a loop 18a (but does not have to comprise a loop 18a) at its distal end portion in the catheter hose. Accordingly, the catheter hose 16a achieves the certain stiffness and elasticity of the elastic wire by glidingly accommodating the latter thereby guiding the endoscope shaft 1 and, at the same time, preventing the endoscope shaft from being bent excessively.

Another possibility of adjusting the stiffness of the catheter hose 16a is to introduce a fluid (a liquid or a gas) into the latter. According to the specific pressure applied to the fluid when introducing the fluid and maintaining the specific pressure in the catheter hose 16a, a certain stiffness of the catheter hose 16a corresponding to the certain pressure is obtained. In this case, when introducing a fluid into the catheter hose 16a, the distal end portion of the catheter hose 16a has to be closed (for example with a plug) so that the catheter hose is sealed fluid tightly. However, as mentioned above, any means for varying the stiffness of the catheter hose 16a known by the prior art may be used.

Referring again to the catheter hose 16a with the guiding wire 17a introduced therein shown in figure 7, corresponding to the power the endoscope shaft 1 is introduced into the canal-shaped cavity by the retained or fixed guiding wire 17a which does not slide in the catheter hose 16a at this time and which projects by the loop 18a thereof beyond the proximal end of the endoscope shaft by a predetermined distance. I.e. the guiding wire performs no relative movement with respect to the endoscope shaft 1 or the catheter hose 16a. However, as mentioned above, the loop 18a of the guiding wire 17a is not obligatory and merely serves to facilitate the propulsion of the endoscope shaft 1.

Upon reaching a curvature and/or bend of the canal-shaped cavity at which the protruding loop 18a of the guiding wire 17a arrives ahead of the endoscope shaft 1, the loop 18a contacts a curved outer wall of the canal-shaped cavity corresponding to the curvature. By virtue of the substantially circular or curved shape of the loop 18a, upon further propulsion it slides along the curved outer wall of the canal-shaped cavity. This ensures that the frictional resistance between the loop 18a of the guiding wire 17a and the curved outer wall is minimized during propulsion of the endoscope shaft 1. Due to the elasticity of the guiding wire 17a, it is bent at the curved outer wall of the canal-shaped cavity by the contact of the loop 18a. Accordingly, due to the elasticity of the guiding wire 17a which is bent in accordance with the curvature of canal-shaped cavity, also the endoscope shaft 1 is bent by the guiding wire 17a, thereby orientating the endoscope shaft 1 according to the curvature of the canal-shaped cavity. Furthermore, the endoscope shaft 1 is prevented from being bent excessively due to the accommodation of the elastic guiding wire 17a in the catheter hose 16a. Since the loop 18a of the guiding wire 17a leads the endoscope shaft 1 upon propulsion, the loop 18a already passes the bend and/or curvature before it is reached by the endoscope shaft and adapts or deforms the flexible endoscope shaft 1 in conformity with the shape of the curvature of the canal-shaped cavity. Thus the guiding wire including the loop 18a which is bent by the course of the curvature takes the lead of the endoscope shaft 1 as well as the orientation thereof which follows the loop 18a.

As already mentioned in the foregoing, during propulsion the endoscope shaft 1 takes up forces in the radial direction as well as in the axial direction thereof which act from the catheter hose 16a via the supports 16b on the endoscope shaft 1 and from the drive means during propulsion. Further the endoscope shaft 1 is bent by virtue of passing the bend or curvature. In order to prevent the high flexible endoscope shaft 1 from excessively curving, the catheter hose 16a acting as guiding means and as stiffness adjusting means is capable of varying the flexural strength thereof. In this way a sufficient flexibility of the endoscope shaft can be brought about and at the same time buckling of the endoscope shaft can be prevented. For example, when using the guiding wire 17a, also the stiffness of the catheter hose 6a accommodating the guiding wire 17a is changed and therefore acts as guiding and stiffness adjusting means. Also it is possible, during propulsion of the endoscope shaft 1, to use guiding wires 17a having a different stiffness and elasticity, respectively, dependent on the degree of the curvature the endoscope shaft 1 has to pass. Accordingly, an accurate stiffness of the endoscope shaft 1 is provided.

Thus, when using the guiding wire 17a with the loop 18a, even portions which are difficult to pass in the canal-shaped cavity, such as e.g. bends and/or curvatures, can easily be passed by means of the guide wire 17a including the loop 18a formed at the leading end thereof.

As one can take from the afore-described drive mode of the continuous drive type endoscope, the loop 18a acts as a kind of leading guide element of the guiding ire 17a with respect to the endoscope shaft 1 which facilitates the movement of the endoscope shaft 1. The design of the loop 18a enables the same to follow the curvatures and/or bends of the canal-shaped cavity.

Upon reaching the location to be examined in the canal-shaped cavity, the guiding wire 17a including the loop 18a can be withdrawn so as to avoid hindering of an appropriate medical treatment/examination carried out by the various means 3 of the endoscope head 2 of the endoscope shaft 1. Accordingly, the respective medical treatment/examination can be carried out without any difficulties.

In the case in which the catheter hose 16a, 16c is only fixedly mounted on the distal end of the endoscope shaft 1 with its end portion, while the remaining part thereof is either guided by the sliding supports or by the groove or canal as shown in figs. 8 (a) and 8 (b), it is also possible to bend the endoscope shaft 1 in a desired direction by controlling the relative movement of the respective catheter hoses 16a, 16c relative to the endoscope shaft 1 due to its flexural rigidity or flexural stiffness as described above. That is, for example, in order to bend the endoscope shaft 1 in a certain direction, one catheter hose 16a, 16c is moved relative to the endoscope shaft 1 in the forward moving direction of the endoscope shaft 1, i.e. the direction of the propulsion of the endoscope shaft 1, while another catheter hose 16a, 16c which is, for example, disposed diametrically opposite to the one catheter hose 16a, 16c, is drawn back, i.e. in a direction opposite to the forward moving direction of the endoscope shaft 1. Accordingly, the endoscope shaft 1 is bent in a desired direction by controlling the relative movements of the respective catheter hoses 16a, 16c. One the one hand, the bending of the catheter hose is caused due to the different relative movements of the respective catheter hoses 16a, 16c with respect to the endoscope shaft 1. One the other hand, since the endoscope shaft 1 has flexural rigidity or flexural stiffness which allows the take up of forces in the longitudinal direction thereof, the endoscope shaft 1 is prevented from being compressed by the relative movement of the catheter hose 16a, 16c moved in the rearward direction and tends to bend in the desired direction.

As it is apparent from the above, the control of the bending of the endoscope shaft 1 is described by using two catheter hoses 16a, 16c, respectively. However, also one or a plurality of catheter hoses can be used in order to control the bending of the endoscope shaft 1. That is, the direction in which the endoscope shaft 1 is to be bended can be controlled the more accurate the more catheter hoses 16a, 16c are disposed in the abovementioned manner on the endoscope shaft 1, respectively.

In a preferred form of this modification, the bending direction of the endoscope shaft 1, i.e. the relative movements of the catheter hoses 16a, 16c, respectively, is controlled by an electronic control unit ECU (not shown in the drawings) which drives/moves or holds each catheter hose 16a, 16c separately by a catheter hose drive means (not shown in the drawings). Accordingly, by driving of the catheter hose drive means, the bending direction of the endoscope shaft 1 can be controlled.

The catheter hose drive means can be any drive means which is able to move the respective catheter hose 16a, 16c in a forward or rearward direction with respect to the moving direction of the endoscope shaft 1 and, furthermore, to hold the respective catheter hose 16a, 16c in a certain position so that no relative movement between the respective catheter hose 16a, 16c and the endoscope shaft 1 occurs. For example, the catheter hose drive means is a roller or a cylinder which is able to wind up/off the catheter hose 16a, 16c corresponding to the relative movement to be carried out by the respective catheter hose 16a, 16c.

Furthermore, in the case of the catheter hoses 16a, 16c which are closed at their distal ends in order to apply a pressure by the introduction of a fluid, the bending of the endoscope shaft 1 can also be controlled. In this case, the catheter hoses 16a, 16c are fixedly arranged along the longitudinal direction of the whole endoscope shaft 1. In more detail, the catheter hoses 16b, 16c are arranged along the endoscope shaft 1 as shown in figs. 7, 8(a) and 8(b) without being movable relative to the endoscope shaft 1 as described above. However, in contrast to the foregoing modification, the endoscope shaft 1 and the catheter hoses 16a, 16c are expandable and compressible in their longitudinal directions. Accordingly, when applying a specific pressure by the introduction of a fluid, the respective catheter hose 16a, 16c begins to expand depending on the elasticity or the material properties of the catheter hose 16a, 16c. Since the respective catheter hose 16a, 16c is fixedly arranged along the endoscope shaft 1 by means of the supports 16b or by an adhesive, for example, the elongation is also transmitted to the endoscope shaft 1 which, in turn, is bent due to the respective elongation. Especially, when the respective catheter hose 16a, 16c is fixedly arranged by the supports 16b as shown in fig. 7, the elongation of the respective catheter hose 16a, 16c between two adjacent supports 16b, respectively, is transmitted by these supports 16b to the endoscope shaft 1. When the respective catheter hose 16a, 16c is directly arranged on the endoscope shaft 1, the elongation of the respective catheter hose 16a, 16c is transmitted by the specific fixation which might be an adhesive, for example. Accordingly, in the same manner as described above, the bending direction of the endoscope shaft is controlled by applying a specific pressure to the respective catheter hose 16a, 16c. Also, the control of applying the pressure to the respective catheter hoses 16a, 16c might be carried out by an electronic control unit as described above.

In order to control the bending movement of the endoscope shaft 1 which is controlled to pass the canal-shaped cavity to be examined, the respective catheter hoses 16a, 16c are preferably elongated within a range of 1,5 to 2cm relative to the endoscope shaft in order to achieve a radius of curvature of the endoscope shaft 1 within the range of 5 to 6cm.

### (Further possible variations)

Finally it is pointed out that the present description and the enclosed figures only have an exemplary character.

Hereinafter some modifications are mentioned:
Basically, the afore-described principle of the alternating propulsion of the endoscope can be started and stopped again in any state of the forward movement of the endoscope. That is to say, the endoscope shaft and the auxiliary means first can be moved into the canal-shaped cavity and the alternating propulsion is only started at a particular position of the cavity which is difficult to pass. After passing this position (e.g. strong bends of the cavity) it is also possible to deactivate and again activate the alternating propulsion during further forward movement.

The shape of the fluid pads has been specified. Any other shape of the fluid pads is possible, however. For instance, the fluid pads could be in the form of a ring surrounding the hose 5 in a plane normal to the longitudinal axis of the hose. The ring should be hollow inside and should be divided into several chambers. Each of the individual chambers formed in this way would then correspond to the function of a fluid pad 6 and, similarly to the fluid pads, pressure would have to be supplied and relieved through feed lines.

Moreover fluid pads 6 can also be completely dispensed with and the flexibility is varied via canal-shaped cavities extending in the longitudinal direction at the outer circumference, at the inner circumference or in the wall of the hose and/or the auxiliary means. Furthermore these canal-shaped cavities could be helically formed at the outer circumference, at the inner circumference or in the wall of the hose and/or the auxiliary means. Likewise a variation of the flexibility of the endoscope shaft 1 can preferably be realized by means of these canal-shaped cavities extending in the longitudinal direction or helically along the endoscope shaft 1 by arranging the canal-shaped cavities at the outer circumference, in the radially outer area or in a working conduit. For varying the flexibility of the auxiliary means and/or the endoscope shaft pressure is applied to or relieved from the fluid or medium provided in the cavities or the physical state of the medium is changed, as will be described later.

The length of the leading end portion of the hose 5 along which the fluid pads 6 are arranged is approximately 50 cm in the first embodiment. However, it is obvious that the length of this leading end portion can be appropriately varied.

Moreover, as described above the fluid pads 6 are arranged at the outer circumference of the hose 5. But it is likewise possible to embed the fluid pads in the hose wall.

Alternatively to the hose 5, a spiral spring could be used. This spiral spring could be used as an auxiliary means with or without fluid pads arranged at the outer circumference.

Furthermore the fluid pads has been arranged in groups of three fluid pads. The number of fluid pads for each group can also be increased, however.

As another modification the fluid pads 6 could vary the flexibility of the hose 5 by the fact that thermal elements are arranged in the respective fluid pads and the fluid pads 6 are filled with a medium which varies its physical state of matter from solid to liquid by heating by means of the thermal elements. In this way, by heating the respective thermal elements, whereby the medium is in a liquid state, the hose 5 could be equipped with a lower flexibility than the endoscope shaft 1. By deactivating the thermal elements the medium in the fluid pads could be solidified, thereby the hose 5 having a lower flexibility than the endoscope shaft 1. The same principle is valid if a medium is used which changes its state from liquid to gaseous when heated by means of the thermal elements.

The variation of flexibility of the hose 5 by means of pressure application or relief of the fluid pads 6 can also be performed in several steps or even continuously. That means the application of pressure to the fluid pads 6 could be controlled such that the higher the pressure applied, the farther the endoscope head 2 is moved forward in a cavity bend while the hose 5 is resting, i.e. the more the deflecting 4 is curved during the forward movement.

In the retracted state of the endoscope shaft 1 (state of Fig. 3a) the leading end (distal end) of the hose 5 ends with the trailing end of the deflecting 4. However, the present invention is not restricted thereto and the leading end of the hose 5 can also end further behind the deflecting 4 in the retracted state of the endoscope shaft 1.

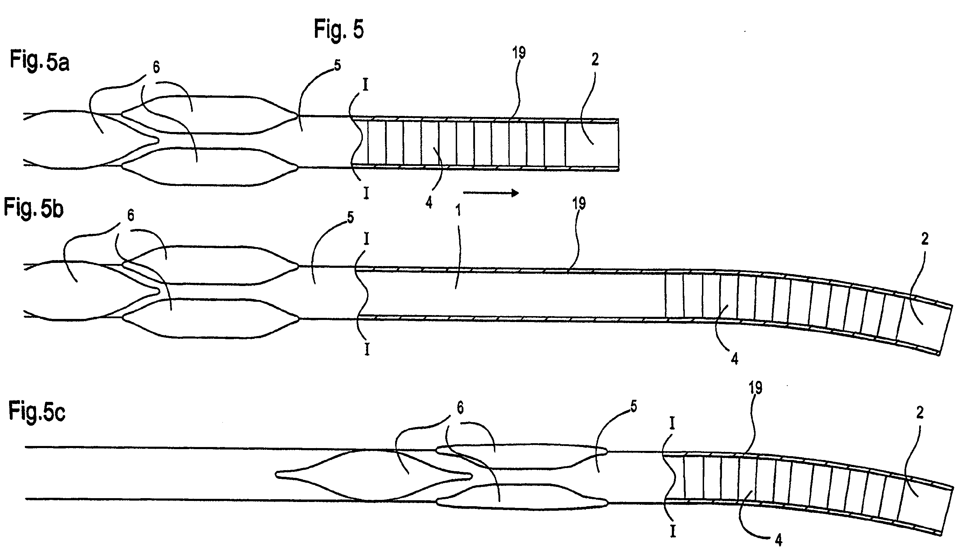

Since the hose 5 is preferably manufactured of a highly flexible EPTFE material, the hose can be strongly expanded without varying its diameter and/or its wall thickness. Therefore, for instance the hose 5 can reach up to the endoscope head 2 and can there be fastened to the same in a fluid-tight manner (e.g. by gluing). This state is shown in Figure 5a, wherein it has to be noted that the endoscope is represented in the longitudinal section in the Figures 5a to 5c right from the line I-I. In the retracted state of the endoscope shaft 5 (state of Figure 5a), the fluid pads 6 are arranged behind the deflecting 4.

If only the endoscope shaft 1 is moved forward, as shown in Figure 5b, while pressure has been applied to the fluid pads and thus they are resting with respect to the cavity wall, an expansion portion 19 of the hose 5 at which no fluid pads 6 are arranged is expanded relative to the hose 5 by the forward movement of the endoscope shaft 1. During this expansion the leading end of the expansion portion 19 is fixed at the endoscope head 2 moving forward and the trailing end of the expansion portion 19 is fixed vis-à-vis the cavity wall by the fluid pads 6 pressing against the cavity wall.

If the hose 5 is moved forward with relieved fluid pads 6, as shown in Figure 5c, the expansion portion 19 of the hose 5 contracts again.

This modification has to be understood as an addition and has the advantage that the clearance between the endoscope shaft 1 and the hose 5 is closed so that neither lubricating stuff can escape from the clearance nor can impurities penetrate the clearance.

The endoscope shaft 1 has been driven, during the propulsion of the hose 5 by the first drive unit 10, at the same speed in the opposite direction by the second drive unit 14.

Alternatively to the opposite drive of the endoscope shaft 1 by the first drive unit 14, the endoscope shaft 1 could also be fastened with respect to the cavity wall to the leading end thereof by means of the deflecting 4 and the first drive unit 14 could be switched to idling so that the drive wheels 13 can rotate freely. Thus the endoscope shaft 1 would likewise be resting vis-à-vis the cavity wall, while the hose 5 is moved forward.

It would be another alternative not to connect the hose 5 to the plate 12. In that case the trailing end of the hose 5 has to be spaced apart from the plate 12 in the state in which the hose 5 is in its leading position with respect to the endoscope head 2 by a distance by which the endoscope shaft 1 moves forward during the movement cycle. With the propulsion of the endoscope shaft 1 in this alternative, the trailing end of the hose 5 would be arranged close to the plate 12 and the drive wheels 11 of the first drive unit 10 would be at rest so that the hose 5 rests with respect to the cavity wall. During propulsion of the hose 5, however, the second drive unit 14 would be at rest so that the drive wheels 13 do not rotate. The bulged shape 15 would not be varied in size during this forward movement of the hose 5 and the trailing end of the hose 5 would move simultaneously with the propulsion into the cavity also away from the plate 12.

The variation of flexibility of the hose 5 by means of the fluid pads 6 has been described. In addition or as an alternative to that, also the flexibility of the endoscope shaft can be varied by fluid pads arranged at the outer circumference of the endoscope shaft or integrated in the outer circumference. The supply lines for these fluid pads of the endoscope shaft would have to extend backwards inside the endoscope shaft to the operating end. The alternating propulsion according to this modification is performed in accordance with the same functioning as described above, wherein the temporarily stiffer element of endoscope shaft and hose supports the simultaneously more flexible element during the forward movement.

Although the first embodiment dispenses, as described above, with the use of a hose 5 including fluid pads 6, also a combination of the first embodiment with a shaft including such fluid pads is possible.

An embodiment not described in more detail works advantageously by advancing the described endoscope shaft, the described hose and the described guiding wire in an alternating manner. In this embodiment the endoscope basically has the afore-described design, wherein, apart from the endoscope shaft, the hose and the guiding wire including pertinent drive units for advancing are provided.

In this embodiment first of all the endoscope shaft is advanced and subsequently the hose is pulled along for supporting the shaft, i.e. it is likewise advanced by the drive unit in a comparable manner. In this way, the endoscope can be brought into the desired position. However, in the case of narrow curves problems of maneuvering the endoscope shaft around the movement of such a narrow curve may arise. In this case, initially the guiding wire possibly including a cage which is easier to maneuver around narrow bends can be advanced. After that the endoscope shaft is guided around the curve by tracking. Consequently, in this case first the guiding wire, possibly including the cage, is advanced and then the endoscope shaft is tracked, i.e. likewise advanced by the drive unit. In a next step the hose can be pushed to stabilize the endoscope shaft. By this alternating movement of the three elements the endoscope can be safely maneuvered even around narrow curves and bends.

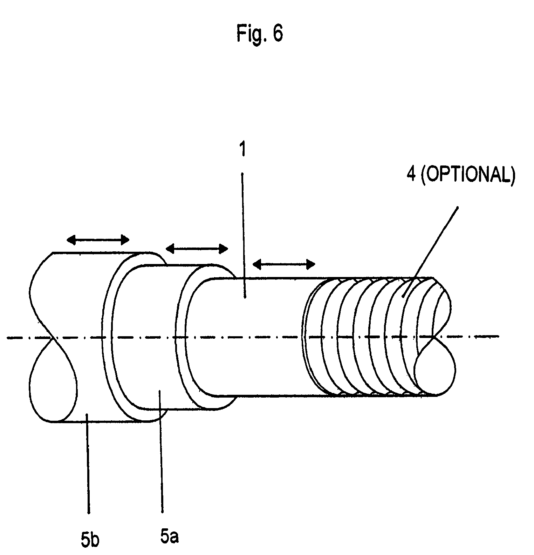

Hereinafter another endoscope design is described by way of Fig. 6.

In this endoscope design the auxiliary means consists of a plurality of (preferably two) hoses which, being movable relative to each other and positioned one inside the other, surround the endoscope shaft at least in portions. The total stiffness of the plurality of hoses is higher than that of the endoscope shaft, wherein the stiffness of preferably each individual hose is lower than that of the endoscope shaft.

If the endoscope shaft is to be driven into a cavity, the plurality of hoses has to be retained in a first step, while the endoscope shaft is moved forward over a certain distance relative to the hoses. The hoses in their entirety serve as a guide for the shaft. After that the shaft is retained, while the hoses are successively pushed forward, i.e. each per se, the shaft having a higher basic stiffness than the individual hoses serving as a guide and accordingly taking up guiding forces.

Finally it is pointed out that the foregoing endoscope designs are mentioned in connection with two drive units 10 and 14. These are not absolutely necessary. It is completely sufficient, for instance, to drive only the shaft by a motor and to retain the auxiliary means temporarily by a clamping means or a brake, for instance.

The tracking of the auxiliary means is then carried out manually while the shaft is retained. It is also possible to perform both movements only manually.

## Claims

1. An endoscope comprising an endoscope shaft (1), having a moveable distal end portion forming a deflecting distal end portion (4) that can be bent in several radii of curvature in response to instructions from the operating end of the endoscope, said moveable distal end portion (4) in turn having an endoscope head (2) at a distal end thereof, said endoscope further comprising at least one catheter hose (16a) adapted to act as a guiding means for guiding the endoscope shaft (1) upon propulsion of the endoscope shaft (1) into a canal-shaped cavity to be examined, wherein the stiffness of said at least one catheter hose (16a) is adjustable, **characterised in that** said endoscope further comprises at least one guiding wire (17a) including a loop (18a) which is formed at the distal end of said guiding wire and protrudes in the axial direction beyond the distal end portion of the endoscope shaft (1).

2. The endoscope according to claim 1 **characterised in that the** at least one catheter hose (16a) is not in contact with the moveable distal end portion (4).

3. The endoscope according to claim 1 or 2, **characterised in that** the at least one catheter hose (16a) is disposed at the outer circumference of the endoscope shaft (1) in the longitudinal direction thereof.

4. The endoscope according to claim 3, **characterised in that** a plurality of catheter hoses (16a) are disposed in equal and/or regular angular distances with respect of the circumferential direction of the endoscope shaft (1) along the circumference to the endoscope shaft (1).

5. The endoscope according to claim 4, **characterised in that** three catheter hoses (16a) are disposed at the outer circumference of the endoscope shaft (1) at angles of 120°.

6. The endoscope according to claim 1 or 2, **characterised in that** a plurality of catheter hoses (16a) are disposed in grooves which are formed on the outer circumference of the endoscope shaft (1) in the longitudinal direction thereof such that only a part of the catheter hoses (16a) protrudes over the outer circumference of the endoscope shaft (1) in the radial direction thereof.

7. The endoscope according to claim 1 or 2, **characterised in that** canals (16c) are formed in the interior of the endoscope shaft (1) extending in the longitudinal direction thereof and disposed close to the outer circumference of the endoscope shaft (1) said canals (16c) being formed by said catheter hoses (16a).

8. The endoscope according to any of the foregoing claims, **characterised in that** the dimension of the loop (18a) is variable.

## Patentansprüche

1. Endoskop umfassend einen Endoskopschaft (1), mit einem beweglichen distalen Endabschnitt das einen biegbaren distalen Endabschnitt (4) formt, welcher in verschiedene Krümmungsradien in Reaktion auf das Betätigungsende des Endoskop biegbar ist, dieser bewegliche distale Endabschnitt (4) weist seinerseits einen Endoskopkopf (2) am distalen Ende auf, weiterhin umfasst dieses Endoskop wenigstens einen Katheterschlauch (16a) welcher angepasst ist, um als Führungsmittel zum Führen des Endoskopfschafts (1) beim Vortrieb des Endoskopschafts (1) in eine zu untersuchende kanalförmige Ausnehmung zu dienen, wobei die Steifigkeit des zumindest einen Katheterschlauches (16a) einstellbar ist, **dadurch gekennzeichnet, dass** dieses Endoskop weiterhin wenigstens ein Führungskabel (17a) mit einer Schlaufe (18a) umfasst, welche am distalen Ende des Führungskabels ausgebildet ist und in axialer Richtung über den distalen Endabschnitt des Endoskopschafts (1) hinausragt.

2. Endoskop nach Anspruch 1, **dadurch gekennzeichnet, dass** der wenigstens eine Katheterschlauch (16a) nicht in Kontakt mit dem beweglichen Endabschnitt (4) steht.

3. Endoskop nach Anspruch 1 oder 2, **dadurch gekennzeichnet, dass** der wenigstens eine Katheterschlauch (16a) in Längsrichtung am Außenumfang des Endoskopschafts (1) angeordnet ist.

4. Endoskop nach Anspruch 3, **dadurch gekennzeichnet, dass** eine Mehrzahl von Katheterschläuchen (16a) in gleichem und/oder gleichbleibendem Winkelabstand in Bezug auf die Umfangsrichtung des Endoskopschafts (1) angeordnet sind.

5. Endoskop nach Anspruch 3, **dadurch gekennzeichnet, dass** drei Katheterschläuche (16a) am Außenumfang des Endoskopschafts (1) in Winkeln von 120° angeordnet sind.

6. Endoskop nach Anspruch 1 oder 2, **dadurch gekennzeichnet, dass** eine Mehrzahl von Katheterschläuche (16a) in Rillen angeordnet sind, welche am Außenumfang in Längsrichtung des Endoskopschafts (1) ausgebildet sind, so dass nur ein Teil der Katheterschläuche (16a) über den Außenumfang in radialer Richtung des Endoskopschafts (1) hinausragt.

7. Endoskop nach Anspruch 1 oder 2, **dadurch gekennzeichnet, dass** sich Kanäle (16c) im Inneren des Endoskopschafts (1) in Längsrichtung erstrecken und diese Kanäle (16c) nahe des Außenumfangs des Endoskopschafts (1) durch die Katheterschläuche (16a) ausgebildet sind.

8. Endoskop nach einem der vorhergehenden Ansprüche, **dadurch gekennzeichnet, dass** die Abmessung der Schlaufe (18a) veränderbar ist.

## Revendications

1. Endoscope comprenant une tige d'endoscope (1), possédant une partie d'extrémité distale mobile formant une partie d'extrémité distale de déflexion (4) qui peut être fléchie selon plusieurs rayons de courbure en réponse à des instructions provenant de l'extrémité opérationnelle de l'endoscope, ladite partie d'extrémité distale mobile (4) possédant quant à elle une tête d'endoscope (2) à une extrémité distale de celle-ci, ledit endoscope comprenant en outre au moins un tube de cathéter (16a) conçu pour servir de moyens de guidage pour guider la tige d'endoscope (1) lors de la propulsion de la tige d'endoscope (1) dans une cavité en forme de canal devant être examinée, dans lequel la rigidité dudit au moins un tube d cathéter (16a) est réglable,
**caractérisé en ce que** ledit endoscope comprend au moins un fil de guidage (17a) incluant une boucle (18a) qui est formée à l'extrémité distale dudit fil de guidage et fait saillie dans la direction axiale au-delà de la partie d'extrémité distale de la tige d'endoscope (1).

2. Endoscope selon la revendication 1, **caractérisé en ce que** l'au moins un tube de cathéter (16a) n'est pas en contact avec la partie d'extrémité distale mobile (4).

3. Endoscope selon la revendication 1 ou 2, **caractérisé en ce que** l'au moins un tube de cathéter (16a) est disposé sur la circonférence externe de la tige d'endoscope (1) dans la direction longitudinale de celle-ci.

4. Endoscope selon la revendication 3, **caractérisé en ce qu'**une pluralité de tubes de cathéter (16a) sont disposés à des distances angulaires égales et/ou régulières par rapport à la direction circonférentielle de la tige d'endoscope (1) le long de la circonférence de la tige d'endoscope (1).

5. Endoscope selon la revendication 4, **caractérisé en ce que** trois tubes de cathéter (16a) sont disposés sur les circonférences externes de la tige d'endoscope (1) à des angles de 120°.

6. Endoscope selon la revendication 1 ou 2, **caractérisé en ce qu'**une pluralité de tubes de cathéter (16a) sont disposés dans des gorges qui sont formées sur la circonférence externe de la tige d'endoscope (1) dans la direction longitudinale de celle-ci, de telle sorte que seule une partie des tubes de cathéter (16a) fait saillie au-dessus de la circonférence externe de la tige d'endoscope (1) dans la direction radiale de celle-ci.

7. Endoscope selon la revendication 1 ou 2, **caractérisé en ce que** des canaux (16c) sont formés à l'intérieur de la tige d'endoscope (1) s'étendant dans la direction longitudinale de celle-ci et disposés près de la circonférence externe de la tige d'endoscope (1), lesdites canaux (16c) étant formés par lesdits tubes de cathéter (16a).

8. Endoscope selon l'une quelconque des revendications précédentes, **caractérisé en ce que** la dimension de la boucle (18a) est variable.
